# EUROPÄISCHE PATENTSCHRIFT

(11) **EP 1 392 390 B1**
(45) Veröffentlichungstag und Bekanntmachung des Hinweises auf die Patenterteilung: **13.06.2012**
(21) Anmeldenummer: 02732683.4
(22) Anmeldetag: 27.04.2002
(51) Int. Cl.: A61M 39/02

(54) **VENTILANORDNUNG FÜR CHIRURGISCHE INSTRUMENTE UND VENTILKORB ZUR AUFNAHME DER VENTILANORDNUNG**
VALVE ARRANGEMENT FOR SURGICAL INSTRUMENTS AND VALVE CAGE FOR ACCOMMODATING THE VALVE ARRANGEMENT
ENSEMBLE SOUPAPE POUR DES INSTRUMENTS CHIRURGICAUX ET LANTERNE DE SOUPAPE RECEVANT L'ENSEMBLE SOUPAPE

(30) Priorität: 02.05.2001 DE 10121356
(43) Veröffentlichungstag der Anmeldung: 03.03.2004
(73) Patentinhaber: Karl Storz GmbH & Co., 78532 Tuttlingen (DE)
(72) Erfinder: STREIFINGER, Wolfgang, 86316 Friedberg-Stätzling (DE); STORM, Gerald, 86153 Augsburg (DE)
(74) Vertreter: Riebling, Peter
(86) Internationale Anmeldenummer: PCT/EP2002/004682
(87) Internationale Veröffentlichungsnummer: WO 2002/087682

(56) Entgegenhaltungen:
- EP-A- 0 283 060
- US-A- 3 402 710
- US-A- 4 181 132
- US-A- 4 475 548
- US-A- 4 634 424
- US-A- 5 207 656
- US-A- 5 356 381
- US-A- 5 527 277
- US-A- 5 549 651
- US-A- 5 931 801
- US-A- 6 146 362

## Beschreibung

Die Erfindung betrifft eine Ventilanordnung für chirurgische Instrumente, insbesondere Trokare, wie sie für minimalinvasive Operationen, z.B. Operationen in Bauchspiegeltechnik, verwendet werden. Des Weiteren ist ein Ventilkorb zur Aufnahme der Ventilanordnung beschrieben.

Aus dem Stand der Technik gehen dazu die US 5,207,656, die US 5,549,651 und die US 4,475,548 hervor. Die US 5,207,656 offenbart ein medizinisches Instrumentenventil mit einem geschäumten Dichtungsbereich, beispielsweise in der Form eines Zugangs für einen Katheter. Das Gehäuse beinhaltet ein Ventil für die Aufnahme und Abdichtung eines länglichen Teiles, welches das Ventil durchdringt. Das Ventil selbst beinhaltet ein elastomeres Trennteil. Dieses Trennteil wird zur Erleichterung der Durchführung und Abdichtung von länglichen Teilen in den Körper hinein vorgestellt. Es besteht aus einem geschlossenzelligen Schaumstoff, in welchem eine Flüssigkeit als Schmiermittel eingeschlossen ist, so dass die Durchführung eines länglichen Körpers durch Herabsetzung der Reibung unterstützt wird. Ein Nachteil dieser Ausführung liegt darin, dass die äußere Oberfläche des Schaumstoffes, also die Seite, in welche ein länglicher Körper in das Ventil hinein eingeführt werden soll, eben ausgebildet ist. Dadurch ist es nicht immer einfach den länglichen Körper für die Durchdringung des Schaumstoffkörpers im Ventil exakt zu positionieren. Es muss also z. T. ein Durchgang durch den Schaumkörper gesucht werden. Dies ist jedoch weder für den Patienten noch für das medizinische Personal erfreulich.

US 5,549,651 beschreibt ein medizinisches Ventil und ein Verfahren für einen Flüssigkeitstranfer. Es handelt sich hierbei um ein intravenöse Leitungssystem, welches mit einem Ventil ausgestattet ist. Das Ventil weist einen Kolben und eine an dessen Vorderseite angeordnete Abdichtung für das Leitungssystem auf. Lediglich die Abdichtung ist aus elastischem Material gefertigt. Sie ist in einer zur Hauptleitung quer angeordneten Führung positioniert. Diese Führung weist im auf die Hauptleitung hin gesehen proximalen Bereich in Längsrichtung zwei sich gegenüberliegende Projektionen auf, die die Abdichtung in diesen beiden Bereichen zusammenquetscht, wenn die Abdichtung in diese Richtung geführt wird. Die Abdichtung weist einen der Länge nach durchgehenden Schlitz auf, der beim Einschieben durch Druck auf die beiden seitlich angeordneten Druckstellen eine ovale Öffnung ausbildet. Durch diese Öffnung hindurch kann nun Flüssigkeit hindurchgeleitet werden. Eine Versorgung mit einem medizinischen Instrument wie bei den zuvor erwähnten Druckschriften ist nicht aber vorgesehen.

Die US 4,475,548 beschreibt einen Ventilkörper. Der Schaumstoff des Ventilkörpers ist vorzugsweise ein chirurgischer Gummi, der aufgeschäumt wurde. Er wird so beschrieben, dass er keine geschlossenen Zellen ausbildet. Für die Druckschrift besteht darin der Vorteil, dass von einer Zelle zur anderen ein Flüssigkeitsaustausch möglich ist. Das bedeutet aber zugleich, dass dadurch keine gasdichte Ausführung möglich ist. Dadurch kann keine absolute Abdichtung zwischen einem hinter einer Körperöffnung liegendem Raum und der Umgebung hergestellt werden. Eine Operation unter abgedichteten Konditionen ist also damit nicht möglich.

Die US 5527277 zeigt alle Merkmale des Oberbegriffs von Anspruch 1.

Trokare sind sowohl in Kunststoffausführung als Einwegartikel als auch in Metallausführung als wiederverwendbare Instrumente bekannt und bestehen im Wesentlichen aus einer Trokarhülse, die in die Bauchdecke eingebracht wird und einer Dichtanordnung in Form eines Ventils, das in einem sogenannten Ventilkorb in Form eines gegenüber der Trokarhülse verbreiterten Gehäuses angeordnet ist, das sich am Zugangsende der Trokarhülse befindet. Trokarhülse und Ventilkorb sind im allgemeinen lösbar miteinander verbunden, um eine leichte Reinigung zu ermöglichen.

Im Ventilkorb ist eine Ventilanordnung angeordnet, welche das eingeführte chirurgische Instrument durchtreten lässt und für eine Abdichtung des Bauchraumes des Patienten gegenüber der Atmosphäre beim Entfernen des Instrumentes, z.B. bei einem Instrumentenwechsel, sorgt. Die Abdichtung des Bauchraumes bei eingeführtem Instrument bewerkstelligt eine dem Instrumentendurchmesser angepasste Gummidichtung oder - kappe.

Derartige Ventilanordnungen benötigen relativ große und schwere Ventilkörbe, die eine unerwünschte "Kopflastigkeit" des Trokars hervorrufen. Ferner sind die gebräuchlichen Ventile schwer zu reinigen, wobei eine unvollständige Reinigung die Gefahr einer Infektion des Patienten birgt.

Die Aufgabe der Erfindung besteht darin, eine Ventilanordnung und einen geeigneten Ventilkorb für Trokare vorzuschlagen, welche in ihrer Dichtwirkung mit den bekannten Ventilanordnungen vergleichbar ist, jedoch preisgünstiger, kleiner und mit geringerem Gewicht ausgeführt werden kann, wobei die medizinischen Instrumente leicht einzuführen sein sollen.

Diese Aufgabe wird durch die Merkmale des unabhängigen Patentanspruchs gelöst.

Erfindungsgemäß besteht der Ventilkörper aus geschlossenzelligem, gasdichten Schaumstoff mit mindestens einem den Ventilkörper durchdringenden Schlitz zum Einführen von im Trokar geführten chirurgischen Instrumenten gemäß Anspruch 1.

Der Ventilkörper ist z.B. als rechteckiger oder zylindrischer Block ausgeführt, der von einer oberen, einer unteren und mindestens einer seitlichen Fläche begrenzt wird. Wichtig ist, dass der Schaumstoff miteinander nichtkommunizierende Hohlräume, sogenannte Vakuolen, aufweist und ist somit als Substanz gasdicht ist.

Die Fläche, die zu dichten ist, ist der innenliegende Ventilschlitz. Die knappe Passung für die einzuführenden Instrumente wird durch die Eigenelastizität des Schaumstoffes erzeugt; dieser führt nach Entfernung des Instrumentes die Dichtflächen automatisch zusammen, so dass sie sich abdichtend berühren. Durch die Eigenelastizität des Schaumstoffes werden die durch den Schlitz gebildeten Ventilflächen aneinandergepresst (Ventilrückstellung). Bei Anlage eines Überdruckes auf der unteren Seite des Ventils reagieren die Vakuolen, indem sie zur Seite ausweichen, sie werden also zusammengedrückt, dadurch drücken Sie auf die Ventilfläche. Je feiner (kleiner) die Vakuolen ausgebildet sind, desto stabiler und steifer wird der Ventilkörper.

Falls Gewebe in den Schlitz gelangt, schließt das Ventil trotzdem, im Gegensatz zu Silikon-Klappenventilen, da es weich vom Schaumstoff umschlossen wird und damit selbst abdichtet. Durch den Schaumstoff wird auch Schmiermittel zur Schmierung der Instrumente vom Bauchraum ferngehalten.

Gemäß der Erfindung ist der Schlitz derart lang ausgebildet, dass er den Ventilkörper in zwei einzelne Hälften unterteilt.

Zwei Hälften lassen sich leichter herstellen und in den Ventilkorb einbringen.

Produktionstechnisch sind das dann z.B. zwei separate Blöcke, die im Ventilgehäuse gegenübergestellt werden. Je breiter der Ventilschlitz und je weicher das Ventilmaterial, desto weniger Druck und somit Reibung liegt am sich darin bewegenden Instrument an; je flacher das Ventil (geringe Bauhöhe), desto geringer ist ebenfalls die Instrumentenreibung.

Die obere Fläche des Ventilkörpers ist mit einer im Wesentlichen keilförmig oder konisch auf den Schlitz zulaufenden Aussparung versehen.

Vorzugsweise wird die Oberfläche der Aussparung zumindest teilweise mit einer im Vergleich zum Schaumstoff glatten und härteren Beschichtung versehen, die aus Kunststoff, metallischem oder keramischem Material bestehen kann.

Durch die glatte Beschichtung der Aussparung wird eine gewisse Gleitfläche und Führung für die einzuführenden Instrumente geschaffen, damit kein Einstechen der Instrumente in den Schaumstoffkörper möglich ist und dieser nicht beschädigt wird.

Die Beschichtung kann durch eine federnde Metallzunge ersetzt werden, die an den Kanten zusätzlich umgebogen ist, um ein Hängenbleiben des einzuführenden oder herauszuziehenden Instruments an der Metallzunge zu verhindern.

Im Bereich der oberen Fläche des Ventilkörpers kann eine zusätzliche Zentrier- und Führungshülse zur Zentrierung und Führung der einzuführenden Instrumente vorgesehen sein. Durch diese Hülse wird ebenfalls einer möglichen Verletzung des Schaumstoffkörpers beim Einführen der Instrumente vorgebeugt. Falls der Schaumstoff beschädigt oder eingerissen wird, bedeutet dies aber nicht unbedingt eine Undichtigkeit des Ventils, da der Schaumstoff eigenelastisch ist und sich selbst wieder in Form bringt.

Es reicht aus, wenn die Länge der Zentrierhülse ca. das 1,5-fache des Instrumentendurchmessers beträgt. Der Durchmesser muss nicht zu genau an den Instrumentendurchmesser angepasst sein, sondern das Instrument muss darin nur lose geführt sein.

In einer weiteren Ausgestaltung weist die untere Fläche des Ventilkörpers schräge, in einem spitzen bis stumpfen Winkel zum Schlitz verlaufende Druckanprallflächen auf. Diese Anschrägung auf der Überdruckseite, wie sie auch beim bekannten Lippenventil in ähnlicher Art vorhanden ist, leitet den Druckvektor derart um, dass die Ventilteile gegeneinandergepresst werden.

Eine stärkere Schließwirkung kann erreicht werden, weil der Ventilkörper mit zwei, den Schlitz seitlich zumindest teilweise umgebenden und in Richtung der unteren Fläche offenen, Hohlräumen versehen ist, hierbei wird je Ventilhälfte ein Hohlraum verwendet.

Die Ventilanordnung ist ein Einwegartikel und wird zusammen mit dem Ventilkorb nach der Benutzung entsorgt bzw. aus dem wiederverwendbaren Ventilkorb entfernt.

Der zur Aufnahme der Ventilanordnung vorgesehene Ventilkorb ist ein Gehäuse, vorzugsweise aus Kunststoff, das in Form und Größe an die Außenabmessungen der Trokarhülse angepasst ist. Das Gehäuse wird abdichtend und lösbar mit einer herkömmlichen Trokarhülse verbunden.

Der Ventilkorb mit Ventilanordnung wird auf den nunmehr zu einem einfachen Rohr reduzierten Mehrwegtrokar aufgesteckt, einmal verwendet und danach entsorgt.

Der Ventilkorb wird an die Standard-Instrumentendurchtrittsdurchmesser, wie 2,5 mm, 5 mm, 10 mm, etc. angepasst.

Im Vergleich zu herkömmlichen Ventilkörben lässt sich der erfindungsgemäße Ventilkorb aufgrund der Ventilanordnung aus Schaumstoff wesentlich kleiner und leichter bauen, da der Ventilöffnungsradius wegfällt und der Schaumstoff sehr leicht ist. Durch die Porigkeit des Schaumstoffes kann die massive Silikongussweise umgangen werden, dadurch wird Masse und Größe eingespart.

Dadurch hat der Trokar keine ausgeprägte Kopflastigkeit mehr und kippt nicht ständig auf die Bauchdecke. Es ergibt sich zudem ein Gewinn an Arbeitshöhe, da das Ventil schlanker zu bauen ist, damit kann das Instrument flacher auf den Bauch gedrückt werden, wodurch sich der Aktionsradius vergrößert.

Durch die Einmal-Verwendung des Ventilkorbes ergibt sich eine Reduktion der Reinigungszeit und dadurch Einsparung an Arbeitszeit. Durch einfachere Reinigung nur der Mehrwegtrokarhülse wird eine bessere Hygiene und Sterilität erzielt.

Die Verbindung zwischen Trokarhülse und Ventilkorb kann als Steckverbindung (Clip), Bajonettverschluss oder Schraubverschluss ausgeführt sein. Wichtig ist, dass die Verbindung zwischen Trokarhülse und Ventilkorb gasdicht ist.

In bekannter Weise kann der Ventilkorb einen Anschluss für die Gaszufuhr aufweisen, der unterhalb des Ventilkörpers angeordnet ist. Die Gaszufuhr kann aber auch an der Trokarhülse angebracht sein.

Ausführungsbeispiele der Erfindung werden nachfolgend anhand der Zeichnungsfiguren beschrieben. Dabei ergeben sich weitere Merkmale, Vorteile und Anwendungsmöglichkeiten der Erfindung.

Es zeigen:
- Figur 1:: einen Schnitt durch ein erstes nicht erfindungsgemäßes Beispiel eines Schaumstoffventils;
- Figur 2:: einen Schnitt durch ein weiteres Beispiel eines Schaumstoffventils;
- Figur 3a:: einen Schnitt durch eine erfindungsgemäße Ausführungsform des Schaumstoffventils;
- Figur 3b:: eine Draufsicht auf Figur 3a;
- Figur 4:: eine schematische Darstellung der Wirkungsweise des Schaumstoffventils;
- Figur 5:: einen Schnitt durch einen Ventilkorb mit exemplarischer Ventilanordnung;
- Figur 6:: einen Schnitt durch einen Ventilkorb mit nicht erfindungsgemäßer Ventilanordnung in Walzenform.

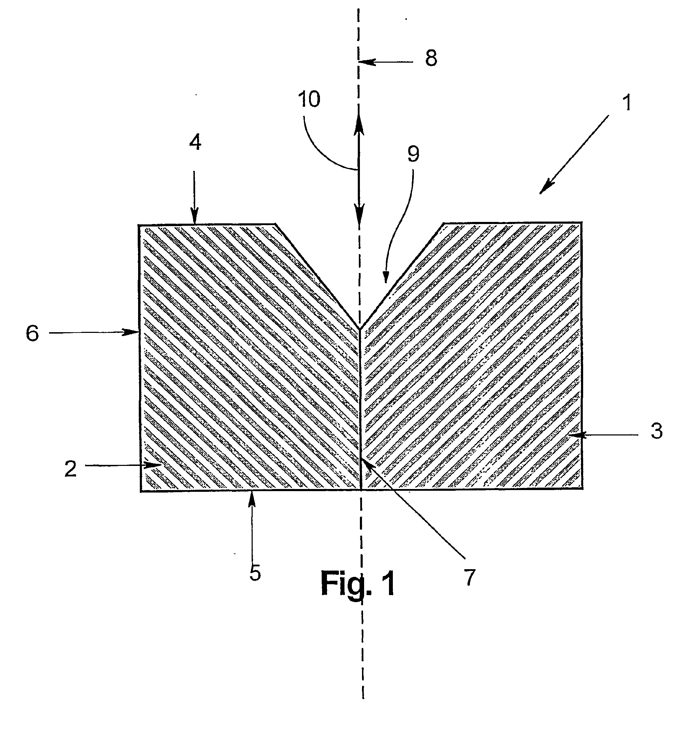

In Figur 1 ist eine erstes nicht erfindungsgemäßes Beispiel einer Ventilanordnung 1 dargestellt. Das Ventil besteht aus einem Ventilkörper 1 aus geschlossenzelligem, gasdichten Schaumstoff, der einen, den Ventilkörper durchdringenden, Schlitz 7 zum Einführen eines im trokargeführten chirurgischen Instruments aufweist. Der Schlitz verläuft in der Zeichnung senkrecht entlang einer Ventilebene und teilt den Ventilkörper 1 in eine linke 2 und rechte Körperhälfte 3. Der Schlitz 7 kann so ausgebildet sein, dass die beiden Körperhälften 2, 3 miteinander verbunden sind, kann aber auch derart ausgebildet sein, dass die beiden Körperhälften 2, 3 getrennte Schaumstoffkörper bilden.

Die obere Fläche 4 des Ventilkörpers 1 weist eine im Wesentlichen keilförmig oder konisch auf den Schlitz zulaufende Aussparung 9 auf, um das Einführen eines chirurgischen Instrumentes in Pfeilrichtung 10 zu erleichtern. Die Seitenflächen 6 und untere Fläche 5 sind an einen Ventilkorb zur Aufnahme der Ventilanordnung angepasst. Der Ventilkorb wird nachfolgend beschrieben.

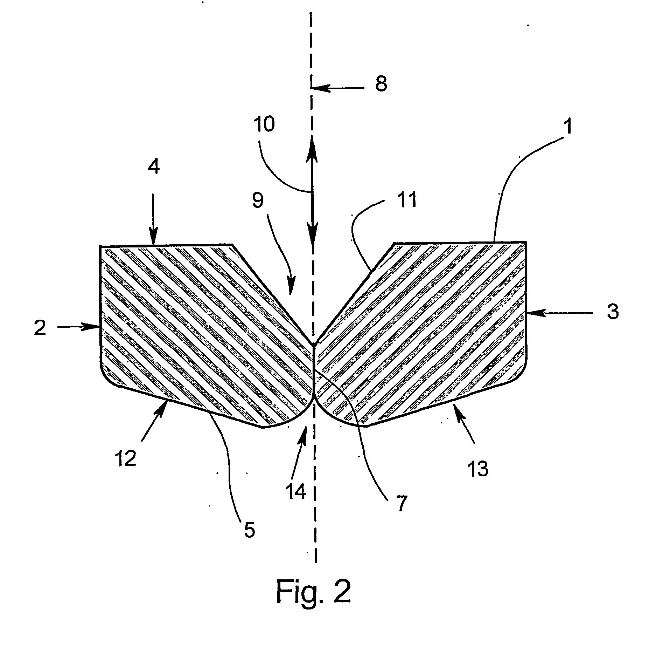

Eine gegenüber Figur 1 abgewandelte exemplarische Form der Ventilanordnung ist in Figur 2 dargestellt. Im Unterschied zu Figur1 ist die Aussparung 9 in der oberen Fläche 4 des Ventilkörpers mit einer glatten Beschichtung 11 versehen, welche z. B. aus Teflon besteht. Diese Beschichtung soll verhindern, dass der Ventilkörper 1 beim Einführen eines chirurgischen Instrumentes in Pfeilrichtung 10 beschädigt wird. Zugleich dient die Beschichtung 11 als Führung für das Instrument in Richtung Ventilschlitz 7.

Die Unterseite 5 des Ventilkörpers ist, ausgehend vom Schlitz 7, mit schrägen Druckprallflächen 12, 13 versehen. An der unteren Fläche 5 des Ventilkörpers 1 herrscht während einer Operation Überdruck, der auf die Druckprallflächen 12, 13 wirkt und die Körperhälften 2, 3 im Bereich des Schlitzes zusammendrückt.

Ebenso wie auf der oberen Fläche 4, kann die untere Fläche 5 im Bereich des Schlitzes 7 mit einer Aussparung 14 versehen sein, um das Herausziehen eines chirurgischen Instrumentes ohne Beschädigung des Schaumstoffkörpers 1 zu ermöglichen.

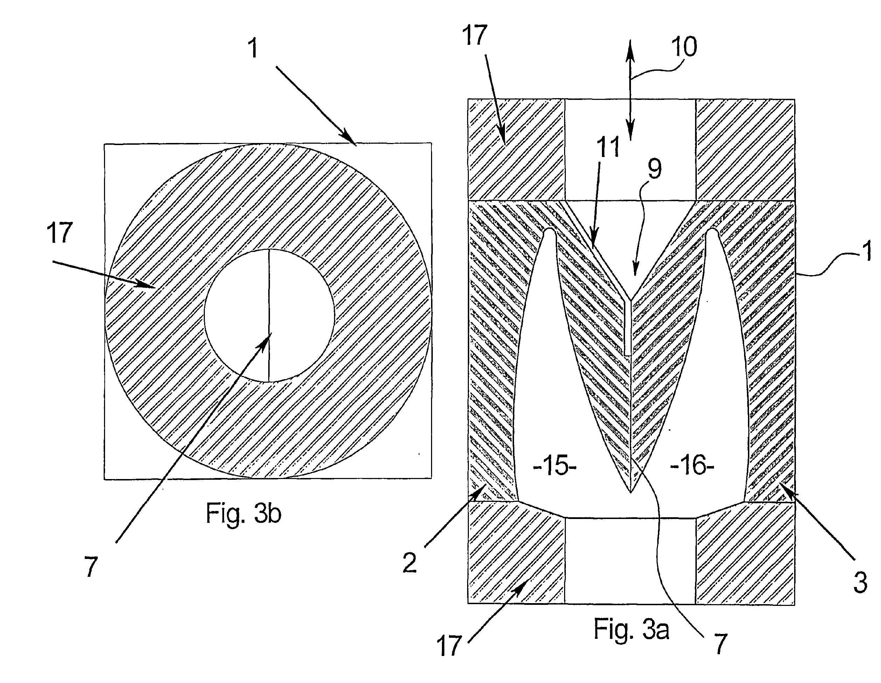

Figuren 3a und 3b zeigen eine erfindungsgemäße Ventilanordnung mit einem Ventilkörper 1, bestehend aus zwei Körperhälften 2, 3, die durch den Schlitz 7 teilweise getrennt sind. In diesem Ausführungsbeispiel ist die Aussparung 9 lediglich einseitig mit einer Beschichtung 11 versehen, die sich teilweise sogar bis in den Schlitz 7 hineinerstreckt. Dadurch soll eine noch bessere Führung eines eingeführten chirurgischen Instrumentes erreicht werden.

Oberhalb und unterhalb des Ventilkörpers 1 sind normale Gummidichtungen oder Schmierdichtungen 17 vorgesehen, welche sich an den Außenumfang eines eingeführten chirurgischen Instrumentes anlegen und dieses in eingeführtem Zustand abdichten und je nach Ausführung zusätzlich schmieren.

Jede Körperhälfte 2, 3 weist einen nach unten offenen Hohlraum 15, 16 auf. Die Hohlräume 15, 16 stehen während der Operation unter Druck und leiten den Druckvektor insbesondere in Richtung Ventilschlitz 7. Die Körperhälften 2, 3 werden daher im Bereich des Schlitzes 7 zusätzlich zusammengepresst, so dass sich eine besonders gute Dichtwirkung erzielen lässt.

In Figur 3b erkennt man insbesondere, dass der Schlitz 7 vorzugsweise nur im mittleren Bereich des Ventilkörpers 1 vorgesehen ist, so dass die beiden Körperhälften 2, 3 in den übrigen Bereich miteinander verbunden sind.

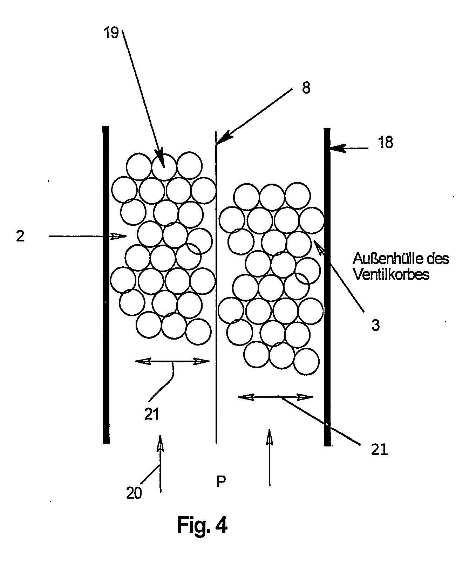

Figur 4 zeigt schematisch die Wirkungsweise des erfindungsgemäßen Ventils, wobei hier die beiden Ventilkörperhälften 2, 3 dargestellt sind, welche aus geschlossenzelligem Schaumstoff bestehen, welcher eine Vielzahl von Vakuolen 19 aufweist. Der Ventilkörper ist in einem Ventilkorb 18 angeordnet und wird seitlich von diesem eingeschlossen. Herrscht nun auf der einen Seite der Ventilanordnung Überdruck, was durch den Pfeil 20 dargestellt ist, so werden die Vakuolen 19 zusammengepresst und dehnen sich zur Seite in Pfeilrichtung 21 aus. Dadurch werden die Körperhälften 2, 3 einerseits an die Wand des Ventilkorbes 18 gepresst und andererseits im Bereich der Ventilebene ebenfalls zusammengepresst, so dass eine hervorragende Abdichtung zwischen Druckseite und Außenatmosphäre gegeben ist.

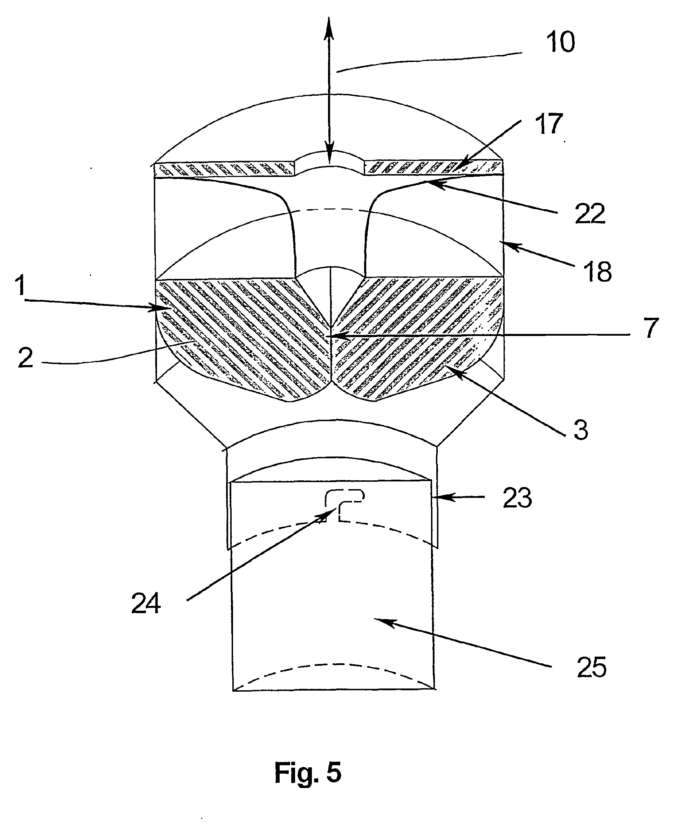

Figur 5 zeigt den Ventilkorb 18, welcher einen Ansatz 23 aufweist und mit einer Trokarhülse 25 verbunden werden kann. Die Verbindung kann z.B. über einen Bajonettverschluss 24 erfolgen. Im Ventilkorb ist die oben beschriebene exemplarische Ventilanordnung in den beiden Körperhälften 2, 3 angeordnet. Oberhalb des Ventilkörpers 1 kann eine Führungshülse 22 zur Führung eines in Pfeilrichtung 10 eingeführten chirurgischen Instrumentes vorgesehen sein. Ferner kann oberhalb der Führungshülse 22 eine, in Zusammenhang mit Figur 3 beschriebene Gummidichtung oder Schmierdichtung 17 angeordnet sein.

Ein derartiger Ventilkorb kann relativ kleindimensioniert sein und ist durch die aus Schaumstoff bestehende Ventilanordnung auch sehr leicht. Der Ventilkorb 18 besteht vorzugsweise aus Kunststoff.

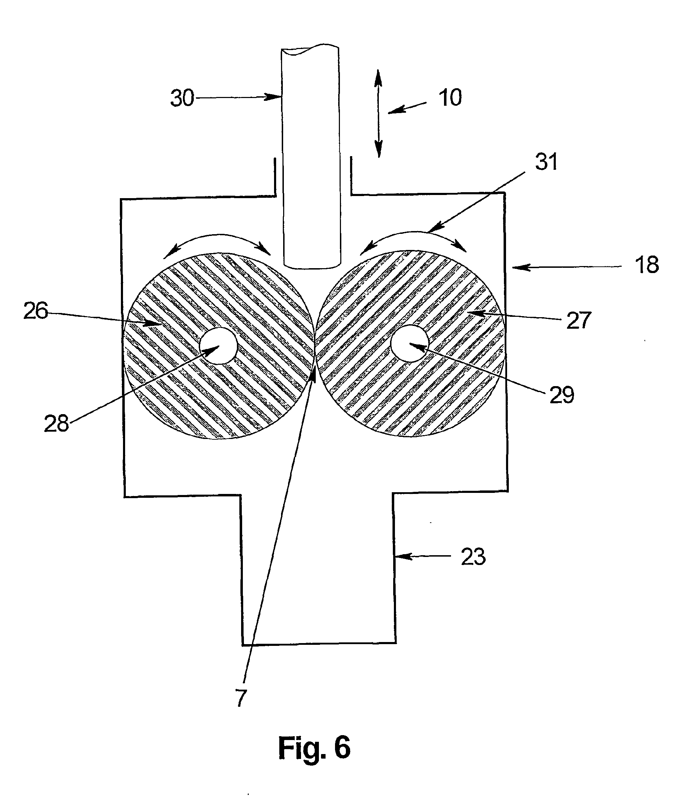

Figur 6 zeigt ein abgewandeltes Ausführungsbeispiel einer in einem Ventilkorb 18 angeordneten nicht erfindungsgemäßen Ventilanordnung. Der Ventilkörper 1 besteht hierbei aus zwei Schaumstoffwalzen 26, 27, welche um jeweils eine Achse 28, 29 drehbar gelagert sind. Die Achsen 28, 29 verlaufen parallel zueinander. Der Ventilschlitz 7 wird durch die Berührungsfläche der beiden Walzen 26, 27 gebildet, die in Pfeilrichtung 31 gegeneinander beweglich sind. Wird nun ein chirurgisches Instrument 30 in Pfeilrichtung 10 eingeführt, so rollen die Walzen 26, 27 an der Oberfläche des Instrumentes 30 ab und gewähren durch die Eigenelastizität des Schaumstoffes eine hervorragende Abdichtung. Andererseits liegen die Schaumstoffwalzen 26, 27 an der Seitenwand des Ventilkörpers 18 an, so dass auch hier eine gute Abdichtung erzielt wird. Die Schaumstoffwalzen 26, 27 bestehen ebenfalls aus im oben beschriebenen, geschlossenzelligen Schaumstoff.

### Zeichnungslegende

- 1: Ventilkörper
- 2: Körperhälfte
- 3: Körperhälfte
- 4: obere Fläche
- 5: untere Fläche
- 6: Seitenfläche
- 7: Schlitz
- 8: Ventilebene
- 9: Aussparung
- 10: Pfeilrichtung
- 11: Beschichtung
- 12: Druckprallfläche
- 13: Druckprallfläche
- 14: Aussparung
- 15: Hohlraum
- 16: Hohlraum
- 17: Schmierdichtung
- 18: Ventilkorb
- 19: Vakuolen
- 20: Pfeilrichtung
- 21: Pfeilrichtung
- 22: Führungshülse
- 23: Ansatz
- 24: Bajonettverschluss
- 25: Trokarhülse (Trokarschaft)
- 26: Schaumstoffwalze
- 27: Schaumstoffwalze
- 28: Achse
- 29: Achse
- 30: Instrument
- 31: Pfeilrichtung

## Patentansprüche

1. Ventilanordnung für ein in einem Trokar geführtes chirurgisches Instrument (30), mit einem Ventilkörper (1), der als Block ausgeführt ist und von einer oberen, einer unteren und mindestens einer seitlichen Fläche (4, 5, 6) begrenzt wird, wobei der Ventilkörper (1) aus zwei Körperhälften (2, 3) besteht, und mindestens ein den Ventilkörper von der oberen Fläche (4) bis zur unteren Fläche (5) durchdringenden Schlitz (7) zum Einführen des im Trokar geführten chirurgischen Instruments (30)vorgesehen ist, wobei die obere Fläche (4) des Ventilkörpers (1) eine im Wesentlichen keilförmig oder konisch auf den Schlitz (7) zulaufende Aussparung (9) aufweist, **dadurch gekennzeichnet, dass** jede Körperhälfte (2, 3) einen nach unten offenen, den Schlitz (7) seitlich zumindest teilweise umgebenden und in Richtung der unteren Fläche offenen Hohlraum (15, 16) aufweist, wobei die Hohlräume (15, 16) während einer Operation unter Druck stehen und den Druckvektor insbesondere in Richtung des Schlitzes (7) leiten, wodurch die Körperhälften (2, 3) im Bereich des Schlitzes (7) zusammengepresst werden, und **dadurch gekennzeichnet, dass** der Ventilkörper aus geschlossenzelligem, gasdichten Schaumstoff besteht.

2. Ventilanordnung nach Anspruch 1, **dadurch gekennzeichnet, dass** die Oberfläche der Aussparung (9) zumindest teilweise mit einer im Vergleich zum Schaumstoff glatten und härteren Beschichtung (11) versehen ist.

3. Ventilanordnung nach Anspruch 2, **dadurch gekennzeichnet, dass** die Beschichtung (11) aus Kunststoff, metallischem oder keramischem Material besteht.

4. Ventilanordnung nach einem der Ansprüche 1 bis 3, **dadurch gekennzeichnet, dass** sie eine im Bereich der oberen Fläche (4) des Ventilkörpers (1) angeordnete Zentrier- und Führungshülse (22) für das einzuführende Instrument (30) aufweist.

5. Ventilanordnung nach einem der Ansprüche 1 bis 4, **dadurch gekennzeichnet, dass** sie als Einwegartikel ausgebildet ist.

6. Kombination einer Ventilanordnung nach einem der Ansprüche 1 bis 5 mit einem Ventilkorb (18) zur Aufnahme der Ventilanordnung, **dadurch gekennzeichnet, dass** der Ventilkorb ein in Form und Größe an die Außenabmessungen des Ventilkörpers (1) angepasstes Gehäuse aufweist, welches abdichtend und lösbar mit einer herkömmlichen Trokarhülse (25) zu verbinden ist.

7. Kombination einer Ventilanordnung mit einem Ventilkorb (18) nach Anspruch 6, **dadurch gekennzeichnet, dass** die Verbindung (24) zwischen dem Gehäuse und der Trokarhülse (25) als Steckverbindung, Bajonettverschluss oder Schraubverschluss ausgeführt ist.

8. Kombination einer Ventilanordnung mit einem Ventilkorb (18) nach einem der Ansprüche 6 oder 7, **dadurch gekennzeichnet, dass** der Ventilkorb (18) einen Anschluss für die Gaszufuhr aufweist, der unterhalb des Ventilkörpers (1) angeordnet ist.

9. Kombination einer Ventilanordnung mit einem Ventilkorb (18) nach einem der Ansprüche 6 bis 8, **dadurch gekennzeichnet, dass** der Ventilkorb (18) aus Kunststoff besteht.

10. Kombination einer Ventilanordnung mit einem Ventilkorb (18) nach einem der Ansprüche 6 bis 9, **dadurch gekennzeichnet, dass** der Ventilkorb (18) als Einwegartikel ausgebildet ist.

## Claims

1. Valve arrangement for a surgical instrument (30) guided in a trocar, with a valve body, which is configured as a block and is limited by an upper, a lower and at least one side face (4, 5, 6), wherein the valve body (1) consists of two body halves (2, 3), and at least one slot (7) passing through the valve body from the upper face (4) to the lower face (5) is provided to introduce the surgical instrument (30) guided in the trocar, the upper face (4) of the valve body (1) having a recess (9) tapering substantially in a wedge shape or conically to the slot (7), **characterised in that** each body half (2, 3) has a cavity (15, 16), which is open at the bottom, laterally surrounds the slot (7), at least partially, and is open in the direction of the lower face (15, 16), wherein the cavities (15, 16) are under pressure during an operation and guide the pressure vector in particular in the direction of the slot (7), so the body halves (2, 3) are pressed together in the region of the slot (7), and **characterised in that** the valve body consists of closed-cell, gas-tight foam.

2. Valve arrangement according to claim 1, **characterised in that** the surface of the recess (9) is at least partially provided with a coating (11), which is smooth and harder in comparison to the foam.

3. Valve arrangement according to claim 2, **characterised in that** the coating (11) consists of plastics material, metallic or ceramic material.

4. Valve arrangement according to any one of claims 1 to 3, **characterised in that** it has a centring and guide sleeve (22) arranged in the region of the upper face (4) of the valve body (1) for the instrument (30) to be introduced.

5. Valve arrangement according to any one of claims 1 to 4, **characterised in that** it is configured as a disposable article.

6. Combination of a valve arrangement according to any one of claims 1 to 5 with a valve cage (18) for receiving the valve arrangement, **characterised in that** the valve cage has a housing, which is adapted to the external dimensions of the valve body (1) with respect to shape and size and which is to be connected to a conventional trocar sleeve (25) in a sealing and detachable manner.

7. Combination of a valve arrangement with a valve cage (18) according to claim 6, **characterised in that** the connection (24) between the housing and the trocar sleeve (25) is configured as a plug connection, bayonet catch or screw closure.

8. Combination of a valve arrangement with a valve cage (18) according to either of claims 6 or 7, **characterised in that** the valve cage (18) has a connection for the gas supply, which is arranged below the valve body (1).

9. Combination of a valve arrangement with a valve cage (18) according to any one of claims 6 to 8, **characterised in that** the valve cage (18) consists of plastics material.

10. Combination of a valve arrangement with a valve cage (18) according to any one of claims 6 to 9, **characterised in that** the valve cage (18) is configured as a disposable article.

## Revendications

1. Ensemble soupape pour un instrument chirurgical (30) guidé dans un trocart, avec un corps de soupape (1) qui est réalisé sous la forme d'un bloc et qui est délimité par une surface supérieure, une surface inférieure et au moins une surface latérale (4, 5, 6), étant précisé que le corps de soupape (1) se compose de deux moitiés de corps (2, 3), qu'il est prévu au moins une fente (7) qui traverse le corps de soupape de la surface supérieure (4) jusqu'à la surface inférieure (5), pour l'introduction de l'instrument chirurgical (30) guidé dans le trocart, et que la surface supérieure (4) du corps de soupape (1) présente un creux (9) qui va en s'effilant vers la fente avec une forme globalement cunéiforme ou conique, **caractérisé en ce que** chaque moitié de corps (2, 3) présente une cavité (15, 16) qui est ouverte vers le bas, qui entoure au moins en partie la fente (7), latéralement, et qui est ouverte en direction de la surface inférieure, étant précisé que les cavités (15, 16), pendant une opération, sont sous pression et dirigent le vecteur de pression en particulier en direction de la fente (7), moyennant quoi les moitiés de corps (2, 3) sont comprimées dans la zone de la fente (7), et **en ce que** le corps de soupape se compose de mousse à pores fermés étanche au gaz.

2. Ensemble soupape selon la revendication 1, **caractérisé en ce que** la surface du creux (9) est pourvue au moins en partie d'un revêtement (11) qui, par rapport à la mousse, est lisse et plus dur.

3. Ensemble soupape selon la revendication 2, **caractérisé en ce que** le revêtement (11) se compose de matière plastique ou d'un matériau métallique ou céramique.

4. Ensemble soupape selon l'une des revendications 1 à 3, **caractérisé en ce qu'**il comporte un manchon de centrage et de guidage (22), pour l'instrument à introduire (30), qui est disposé dans la zone de la surface supérieure (4) du corps de soupape (1).

5. Ensemble soupape selon l'une des revendications 1 à 4, **caractérisé en ce qu'**il est conçu comme un article à usage unique.

6. Combinaison d'un ensemble soupape selon l'une des revendications 1 à 5, avec un boîtier de soupape (18) destiné à recevoir l'ensemble soupape, **caractérisée en ce que** le boîtier de soupape comporte une enveloppe dont la forme et la taille sont adaptées aux dimensions extérieures du corps de soupape (1) et qui peut être reliée de manière étanche et amovible à un trocart (25) classique.

7. Combinaison d'un ensemble soupape avec un boîtier de soupape (18) selon la revendication 6, **caractérisée en ce que** la liaison (24) entre l'enveloppe et le trocart (25) est réalisée sous la forme d'un raccord enfichable, d'un joint à baïonnette ou d'une fermeture à vis.

8. Combinaison d'un ensemble soupape avec un boîtier de soupape (18) selon l'une des revendications 6 ou 7, **caractérisée en ce que** le boîtier de soupape (18) comporte un raccordement pour l'amenée de gaz, qui est disposé au-dessous du corps de soupape (1).

9. Combinaison d'un ensemble soupape avec un boîtier de soupape (18) selon l'une des revendications 6 à 8, **caractérisée en ce que** le boîtier de soupape (18) est en matière plastique.

10. Combinaison d'un ensemble soupape avec un boîtier de soupape (18) selon l'une des revendications 6 à 9, **caractérisée en ce que** le boîtier de soupape (18) est conçu comme un article à usage unique.
